# NEW EUROPEAN PATENT SPECIFICATION

(11) **EP 1 450 799 B2**
(45) Date of publication and mention of the opposition decision: **11.07.2018**
(45) Mention of the grant of the patent: 15.11.2006
(21) Application number: 02786842.1
(22) Date of filing: 03.12.2002
(51) Int. Cl.: A61K 31/44, A61K 31/535, A61K 31/65, A61K 31/435, A61K 31/505, A61K 31/47, A61P 35/00

(54) **ARYL UREA COMPOUNDS IN COMBINATION WITH OTHER CYTOSTATIC OR CYTOTOXIC AGENTS FOR TREATING HUMAN CANCERS**
ARYLHARNSTOFF-VERBINDUNGEN IN KOMBINATION MIT ANDEREN ZYTOSTATISCH ODER ZYTOTOXISCH WIRKSAMEN STOFFEN ZUR BEHANDLUNGEN MENSCHLICHER KREBSERKRANKUNGEN
COMPOSES DE TYPE UREE ARYLE COMBINES A D'AUTRES AGENTS CYTOSTATIQUES OU CYTOTOXIQUES ET SERVANT A TRAITER DES CANCERS HUMAINS

(30) Priority: 03.12.2001 US 334609 P
(43) Date of publication of application: 01.09.2004
(60) Divisional application: 06023696.5
(73) Proprietor: Bayer HealthCare LLC, Whippany, NJ 07981 (US)
(72) Inventor: CARTER, Christopher, A., Guilford, CT 06437 (US); DUMAS, Jacques, Orange, CT 06477 (US); GIBSON, Neil, East Northport, NY 11731 (US); HIBNER, Barbara, Clayton, CA 94517 (US); HUMPHREY, Rachel, W., Woodbridge, CT 06525 (US); TRAIL, Pamela, Madison, CT 06443 (US); VINCENT, Patrick, W., Cheshire, CT 06410 (US); ZHAI, Yifan, Guilford, CT 06437 (US); RIEDL, Bernd, 42329 Wuppertal (DE); KHIRE, Uday, Hamden, CT 06518 (US); LOWINGER, Timothy, B., 203, 5-7 Chitose-Cho, Hyogo 662-0046 (JP); SCOTT, William, J., Guilford, CT 06437 (US); SMITH, Roger, A., Madison, CT 06443 (US); WOOD, Jill, E., North Haven CT 06473 (US); MONAHAN, Mary-Katherine, Hamden, CT 06517 (US); NATERO, Reina, Hamden, CT 06518 (US); RENICK, Joel, Milford, CT 06460 (US); SIBLEY, Robert, N., North Haven, CT 06473 (US)
(74) Representative: Weickmann & Weickmann PartmbB
(86) International application number: PCT/US2002/038439
(87) International publication number: WO 2003/047579

(56) References cited:
- WO-A-00/42012
- WO-A-00/56331
- WO-A-98/52559
- WO-A-99/32106
- WO-A-99/32455
- WO-A1-01/12188
- WO-A2-01/80843
- WO-A2-02/14311
- WO-A2-02/44156
- DATABASE MEDLINE [Online] June 1993 (1993-06) IWADATE Y ET AL: "[Intra-arterial ACNU, CDDP chemotherapy for brain metastases from lung cancer: comparison of cases with and without intra-arterial mannitol infusion]" Database accession no. NLM8336809 XP002233466 & NO SHINKEI GEKA. NEUROLOGICAL SURGERY. JAPAN JUN 1993, vol. 21, no. 6, June 1993 (1993-06), pages 513-518, ISSN: 0301-2603
- GEIGER T ET AL: "Antitumor activity of a C-raf antisense oligonucleotide in combination with standard chemotherapeutic agents against various human tumors transplanted subcutaneously into nude mice." CLINICAL CANCER RESEARCH: AN OFFICIAL JOURNAL OF THE AMERICAN ASSOCIATION FOR CANCER RESEARCH. UNITED STATES JUL 1997, vol. 3, no. 7, July 1997 (1997-07), pages 1179-1185, XP001145779 ISSN: 1078-0432
- CUNNINGHAM C CASEY ET AL: "A phase I trial of H-ras antisense oligonucleotide ISIS 2503 administered as a continuous intravenous infusion in patients with advanced carcinoma." CANCER, vol. 92, no. 5, 1 September 2001 (2001-09-01), pages 1265-1271, XP002232130 ISSN: 0008-543X
- RIEDL B ET AL: "Potent Raf kinase inhibitors from the diphenylurea class: Structure activity relationships." PROCEEDINGS OF THE AMERICAN ASSOCIATION FOR CANCER RESEARCH ANNUAL, vol. 42, March 2001 (2001-03), page 923 XP001145518 92nd Annual Meeting of the American Association for Cancer Research;New Orleans, LA, USA; March 24-28, 2001, March, 2001 ISSN: 0197-016X
- STRUMBERG DIRK ET AL: "Phase I and pharmacokinetic study of the Raf kinase inhibitor bay 43-9006 in patients with locally advanced or metastatic cancer." PROCEEDINGS OF THE AMERICAN ASSOCIATION FOR CANCER RESEARCH ANNUAL, vol. 42, March 2001 (2001-03), page 543 XP001145481 92nd Annual Meeting of the American Association for Cancer Research;New Orleans, LA, USA; March 24-28, 2001, March, 2001 ISSN: 0197-016X
- Römpp Chemie Lexikon, CD- Vers. 1.0 "Zytostatika" 1995
- Wikipedia "Zytostatika"
- Römpp Chemie Lexikon, CD- Vers. 1.0 "Krebs" 1995
- Römpp Chemie Lexikon, CD- Vers. 1.0 "Chemotherapie, (-therapeutika)" 1995
- Wikipedia "Topoisomerasehemmer"
- Römpp Chemie Lexikon, CD- Vers. 1.0 "Camptothecin" 1995
- Doklady Bogarskoi Akademii Nauk, vol. 37, n°1 1984
- DE VITA V. ET AL: 'Cancer: Principles & Practice of Oncology', vol. 5TH EDIT, part 17 1997, LIPPINCOTT-RAVEN PUBLISHERS, PHILADELPHIA pages 333 - 347
- BAUMANN B. ET AL: 'Raf induces NF-kB by membrane shuttle kinase MEKK1, a signaling pathway critical for transformation' PNAS vol. 97, no. 9, 25 April 2000, pages 4615 - 4620
- website Wikipedia: Zytotoxirität
- MONIA B.P. ET AL: 'Antitumor activity of a phosphorothioate antisense oligodeoxynucleotide targeted against C-raf kinase' NATUREMEDICINE vol. 2, no. 6, June 1996, pages 668 - 675
- KOLCH W. ET AL: 'Raf-1 protein kinase is required for growth of induced NIH/3T3 cells' NATURE vol. 349, 31 January 1991, pages 426 - 428
- TENNEY S. ET AL: 'Molecular Cell Biology', vol. 4TH EDIT, 2000, W.H. FREEMAN AND COMPANY pages 878 - 879
- LEICHT D.T. ET AL: 'Raf kinases: Function, regulation and role in human cancer' BIOCHIM. BIOPHYS. ACTA. vol. 1773, no. 8, August 2007, pages 1196 - 1212

## Description

### Cross-Reference to Related Application

This is application claims priority to provisional application Serial No. 60/334,609, filed December 3, 2001.

### Field of the Invention

This invention relates to aryl urea compounds in combination with cytotoxic or cytostatic agents and their use in treating raf kinase mediated diseases such as cancer, wherein said aryl urea compound is a tosylate salt of N-(4-chloro-3-(trifluoromethyl)phenyl-N'-(4-(2-(N-methylcarbamoyl)-4-pyridyloxy)phenyl)urea (compound A).

### Background of the Invention

The p21 oncogene, ras, is a major contributor to the development and progression of human solid cancers and is mutated in 30% of all human cancers (Bolton et al. Ann. Re. Med. Chem. 1994, 29, 165-174; Bos. Cancer Res. 1989, 49, 4682-9). In its normal, unmutated form, the ras protein is a key element of the signal transduction cascade directed by growth factor receptors in almost all tissues (Avruch et al. Trends Biochem. Sci. 1994, 19, 279-83). Biochemically, ras is a guanine nucleotide binding GTPase protein that cycles between a GTP-bound activated and a GDP-bound inactive form. It's endogenous GTPase activity is strictly self-regulated and is also controlled by other regulatory proteins. The endogenous GTPase activity of mutations is reduced. Therefore, the protein delivers constitutive growth signals to downstream effectors such as the enzyme raf kinase. This leads to the cancerous growth of the cells which carry these mutants (Magnuson et al. Semin. Cancer Biol. 1994, 5, 247-53). It has been shown that inhibiting the effect of active ras by inhibiting the raf kinase signaling pathway via administration of deactivating antibodies to raf kinase or by co-expression of dominant negative raf kinase or dominant negative MEK, the substrate of raf kinase, leads to the reversion of transformed cells to the nomial growth phenotype (see: Daum et al. Trends Biochem. Sci. 1994, 19, 474-80; Friedman et al. J. Biol. Chem. 1994, 269, 30105-8; Kocj et al. Nature 1991, 349, 426-28). These references have further indicated that inhibition of raf expression by antisense RNA blocks cell proliferation in membrane-associated oncogenes. Similarly, inhibition of raf kinase (by antisense oligodeoxynucleotides) has been correlated in vitro and in vivo with inhibition of the growth of a variety of human cancer types (Monia et al., Nat. Med. 1996, 2, 668-75).

Therefore, compounds which can act as raf kinase inhibitors represent an important group of chemotherapeutic agents for use in the treatment of a variety of different cancer types.

### Summary of the Invention

Generally, it is the overall object of the present invention to provide cytotoxic and/or cytostatic agents in combination with an aryl urea compound raf kinase inhibitors which is a tosylate salt of N-(4-chloro-3-(trifluoromethyl)phenyl-N'-(4-(2-(N-methylcarbamoyl)-4-pyridyloxy)phenyl)urea and which will serve to (1) yield better efficacy in reducing the growth of a tumor or even eliminate the tumor as compared to administration of either agent alone, (2) provide for the administration of lesser amounts of the administered chemotherapeutic agents, (3) provide for a chemotherapeutic treatment that is well tolerated in the patient with fewer deleterious pharmacological complications than observed with single agent chemotherapies and certain other combined therapies, (4) provide for treating a broader spectrum of different cancer types in mammals, especially humans, (5) provide for a higher response rate among treated patients, (6) provide for a longer survival time among treated patients compared to standard chemotherapy treatments, (7) provide a longer time for tumor progression, and/or (8) yield efficacy and tolerability results at least as good as those of the agents used alone, compared to known instances where other cancer agent combinations produce antagonistic effects.

### Brief Description of the Figures

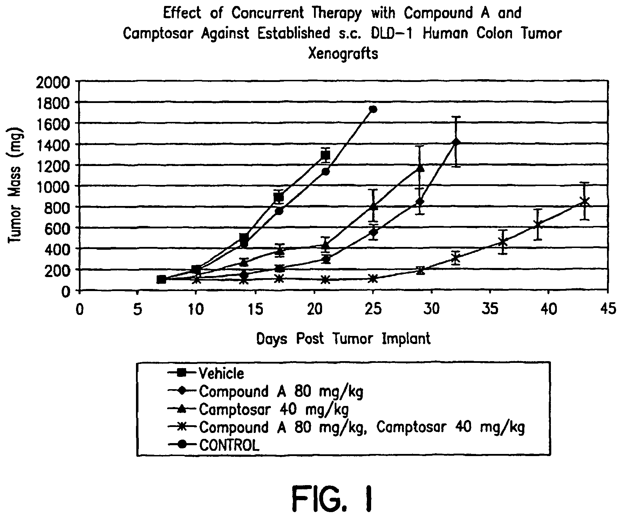

Figure 1 shows the response of established s.c. DLD-1 human colon tumor xenografts to Compound A and Camptosar alone and in combination.

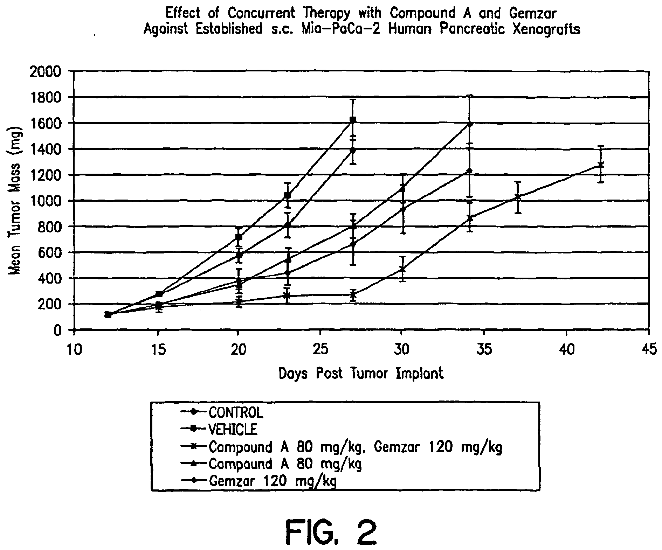

Figure 2 shows the response of established s.c. MiaPaCa-2 human pancreatic tumor xenografts to Compound A and Gemzar alone and in combination.

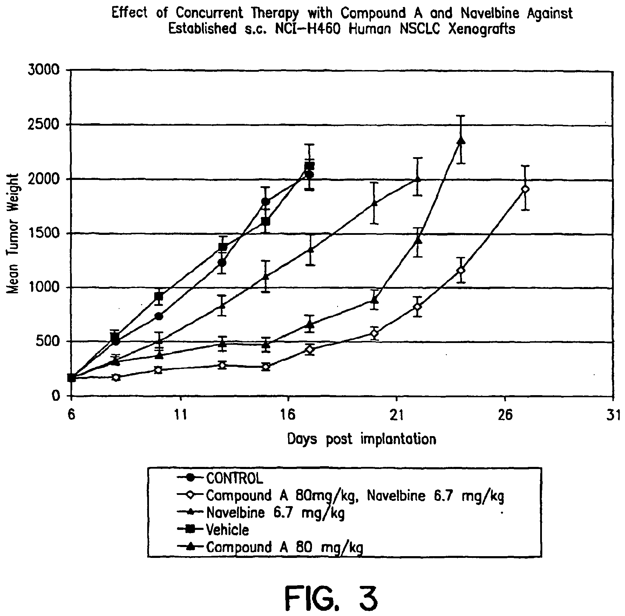

Figure 3 shows the response of established s.c. NCI-H460 human NSCLC tumor xenografts to Compound A and Navelbine alone and in combination;

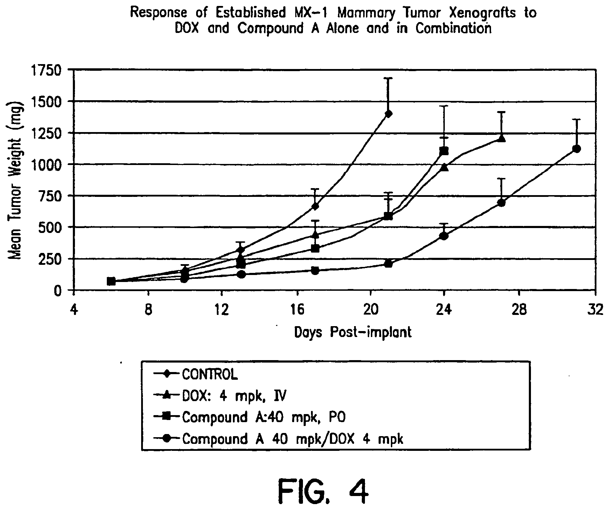

Figure 4 shows the response of established MX-1 mammary tumor xenografts to Compound A and DOX alone and in combination.

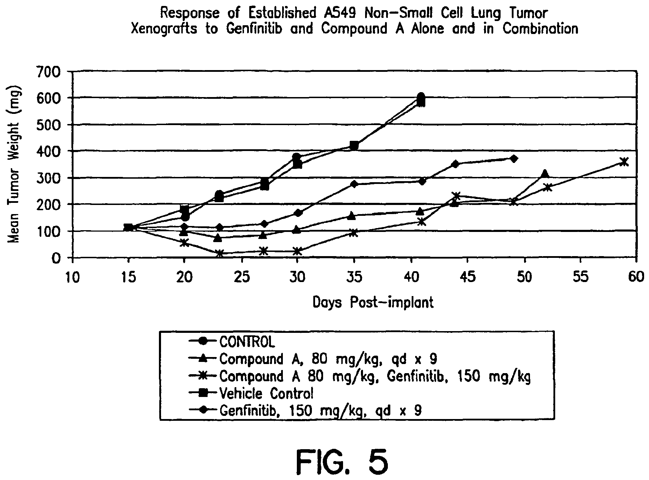

Figure 5 shows the response of established A549 non-small cell lung tumor xenografts to Compound A and Gefitinib alone and in combination.

### Detailed Description of the Invention

The present invention relates to combination comprising an aryl urea compound which is a tosylate salt of N(4-chloro-3-(trifluoromethyl)phenyl-N'-(4-(2-(N-methylcarbamoyl)-4-pyridyloxy)phenyl urea with at least one other chemotherapeutic (a) cytotoxic agent or (b) cytostatic agent or pharmaceutically acceptable salts of component (a) or (b).

The invention also relates to a pharmaceutical preparation which comprises (1) quantities of (a) an aryl urea compound which is Compound A (defined below) and (b) at least one other cytotoxic or cytostatic agent in amounts which are jointly effective for treating a cancer, where component (b) can also be present in the form of a pharmaceutically acceptable salt if at least one salt-forming group is present, with (2) one or more pharmaceutically acceptable carrier molecules.

Also disclosed is a method for treating a cancer that can be treated by administration of compound A that targets raf kinase and at least one other chemotherapeutic agent which is a cytotoxic or cytostatic agent. The aryl urea compound and cytotoxic or cytostatic agent are to be administered to a mammal in quantities which together are therapeutically effective against proliferative diseases, including but not limited to colon, gastric, lung, pancreatic, ovarian, prostate, leukemia, melanoma, hepatocellular, renal, head and neck, glioma, and mammary cancers. Thus, the aryl urea compound is effective for raf kinase-mediated cancers. However, these compounds are also effective for cancers not mediated by raf kinase.

In a preferred embodiment, the cytotoxic or cytostatic agent of the present invention includes but is not limited to irinotecan, vinorelbine, gemcitabine, gefitinib, paclitaxel, taxotere, doxorubicin, cisplatin, carboplatin, BCNU, CCNU, DTIC, melphalan, cyclophosphamide, ara A, ara C, etoposide, vincristine, vinblastine, actinomycin D, 5-fluorouracil, methotrexate, herceptin, and mitomycin C.

The present invention describes methods for treating a cancer in a mammal, especially a human patient, comprising administering an aryl urea compound which is a tosylate salt of N-(4-chloro-3-trifluoromethyl)phenyl)-N'-(4-(2-(N-methylcarbamoyl-4-pyridyloxy)phenyl)urea in combination with a cytotoxic or cytostatic chemotherapeutic agent including but not limited to DNA topoisomerase I and II inhibitors, DNA intercalators, alkylating agents, microtubule disruptors, hormone and growth factor receptor agonists or antagonists, other kinase inhibitors and antimetabolites.

The present invention describes a method for treating a cancer in a mammal, especially a human patient, comprising administering an aryl urea compound which is a tosylate salt of N-(4-chloro-3-trifluoromethyl)phenyl)-N'-(4-(2-(N-methylcarbamoyl-4-pyridyloxy)phenyl)urea in combination with irinotecan.

The present invention describes a method for treating a cancer in a mammal, especially a human patient, comprising administering an aryl urea compound which is a tosylate salt of N-(4-chloro-3-trifluoromethyl)phenyl)-N'-(4-(2-(N-methylcarbamoyl-4-pyridyloxy)phenyl)urea in combination with paclitaxel.

The present invention describes a method for treating a cancer in a mammal, especially a human patient, comprising administering an aryl urea compound which is a tosylate salt of N-(4-chloro-3-trifluoromethyl)phenyl)-N'-(4-(2-(N-methylcarbamoyl-4-pyridyloxy)phenyl)urea in combination with vinorelbine.

The present invention describes a method for treating a cancer in a mammal, especially a human patient, comprising administering an aryl urea compound which is a tosylate salt of N-(4-chloro-3-trifluoromethyl)phenyl)-N'-(4-(2-(N-methylcarbamoyl-4-pyridyloxy)phenyl)urea in combination with gefitinib.

The present invention describes a method for treating a cancer in a mammal, especially a human patient, comprising administering an aryl urea compound which is a tosylate salt of N-(4-chloro-3-trifluoromethyl)phenyl)-N'-(4-(2-(N-methylcarbamoyl-4-pyridyloxy)phenyl)urea in combination with doxorubicin.

The present invention describes a method for treating a cancer in a mammal, especially a human patient, comprising administering an aryl urea compound which is a tosylate salt of N-(4-chloro-3-trifluoromethyl)phenyl)-N'-(4-(2-(N-methylcarbamoyl-4-pyridyloxy)phenyl)urea in combination with gemcitabine.

The methods can be used to treat a variety of human cancers, including but not limited to pancreatic, lung, colon, ovarian, prostate, leukemia, melanoma, hepatocellular, renal, head and neck, glioma, and mammary carcinomas.

A method is disclosed for administering the chemotherapeutic agents, including the aryl urea compound which is a tosylate salt of N-(4-chloro-3-trifluoromethyl)phenyl)-N'-(4-(2-(N-methylcarbamoyl-4-pyridyloxy)phenyl)urea and the cytotoxic and cytostatic agents, to the patient by oral delivery or by intravenous injection or infusion.

In another preferred embodiment, the composition comprising the aryl urea compound which is a tosylate salt of N-(4-chloro-3-trifluoromethyl)phenyl)-N'-(4-(2-(N-methylcarbamoyl-4-pyridyloxy)phenyl)urea or the cytotoxic or cytostatic agent can be administered to a patient in the form of a tablet, a liquid, a topical gel, an inhaler or in the form of a sustained release composition.

In one embodiment of the invention, the aryl urea compound which is a tosylate salt of N-(4-chloro-3-trifluoromethyl)phenyl)-N'-(4-(2-(N-methylcarbamoyl-4-pyridyloxy)phenyl)urea can be administered simultaneously with a cytotoxic or cytostatic agent to a patient with a cancer, in the same formulation or, more typically in separate formulations and, often, using different administration routes. Administration can also be sequentially, in any order.

In a preferred embodiment, the aryl urea compound which is a tosylate salt of N-(4-chloro-3-trifluoromethyl)phenyl)-N'-(4-(2-(N-methylcarbamoyl-4-pyridyloxy)phenyl)urea can be administered in tandem with the cytotoxic or cytostatic agent, wherein the aryl urea compound can be administered to a patient once or more per day for up to 28 consecutive days with the concurrent or intermittent administration of a cytotoxic or cytostatic agent over the same total time period.

In another preferred embodiment of the invention, the aryl urea compound which is a tosylate salt of N-(4-chloro-3-trifluoromethyl)phenyl)-N'-(4-(2-(N-methylcarbamoyl-4-pyridyloxy)phenyl)urea can be administered to a patient at an oral, intravenous, intramuscular, subcutaneous, or parenteral dosage which can range from about 0.1 to about 300 mg/kg of total body weight.

In another preferred embodiment, the cytotoxic or cytostatic agent can be administered to a patient at an intravenous, intramuscular, subcutaneous, or parenteral dosage which can range from about 0.1 mg to 300 mg/kg of patient body weight.

The aryl urea compound is a tosylate salt of N-(4-chloro-3-(trifluoromethyl)phenyl)-N'-(4-(2-(N-methylcarbamoyl)-4-pyridyloxy)phenyl)urea. The scaleable synthesis of the aryl urea compound is disclosed in Organic Process Research and Development (2002), Vol.6, Issue #6, 777-781, and copending patent application serial no. 09/948,915 filed September 10, 2001.

Further, the invention describes a method of inhibiting proliferation of cancer cells comprising contacting cancer cells with a pharmaceutical preparation or product of the invention, especially a method of treating a proliferative disease comprising contacting a subject, cells, tissues or a body fluid of said subject, suspected of having a cancer with a pharmaceutical composition or product of this invention.

This invention also relates to compositions containing both the aryl urea compound and the other cytotoxic or cytostatic agents, in the amounts of this invention. This invention further relates to kits comprising separate doses of the two mentioned chemotherapeutic agents in separate containers. The combinations of the invention can also be formed in vivo, e.g., in a patient's body.

The term "cytotoxic" refers to an agent which can be administered to kill or eliminate a cancer cell. The term "cytostatic" refers to an agent which can bo administered to restrain tumor proliferation rather than induce cytotoxic cytoreduction yielding an elimination of the cancer cell from the total viable cell population of the patient. The chemotherapeutic agents described herein, e.g., irinotecan, vinorelbine, gemcitabine, doxorubicin, and paclitaxel are considered cytotoxic agents. Gefitinib is considered a cytostatic agent. These cytotoxic and cytostatic agents have gained wide spread use as chemotherapeutics in the treatment of various cancer types and are well known.

Irinotecan (CPT-11) is sold under the trade name of Camptosar® by Pharmacia & Upjohn Co., Kalamazoo, MI. Irinotecan is a camptothecin ortopoisomerase I inhibitor. While not being bound by a theory, it is believed that by blocking this enzyme in cells, damage occurs when the cell replicates, and the cancer growth is thus controlled. The cytotoxic effect is believed due to double-stranded DNA damage produced during DNA synthesis when replication enzymes interact with the tertiary complex formed by topoisomerase I, DNA, and either Irinotecan or SN-38 (its active metabolite). Conversion of irinotecan to SN-38 is believed to occur in the liver. Irinotecan is typically administered by injection or via i.v. infusion.

Vinorelbine (Vinorelbine tartrate) has the molecular formula C₄₅H₅₄N₄O₈·2C₄H₆O₆ with a molecular weight of 1079.12 and is sold under the tradename of Navelbine® by Glaxo SmithKline, Research Triangle Park. Vinorelbine is a semi-synthetic vinca alkaloid with antitumor activity. The chemical name is 3',4'-didehydro-4'deoxy-C-norvincaleukob-lastine [R-(R,R)-2,3-dihydroxybutanedioate (1:2)(salt)]. While not bound by a theory, the antitumor activity of vinorelbine is believed to be due primarily to inhibition of mitosis at the metaphase stage through its interaction with tubulin. Vinorelbine may also interfere with: 1) amino acid, cyclic AMP, and glutathione metabolism, 2) calmodulin dependent Ca++ transport ATPase activity, 3) cellular respiration, and 4) nucleic acid and lipid biosynthesis. Vinorelbline is typically administered by intravenous injection (i.v.) or by other appropriate infusion techniques. Vinorelbine is typically prepared in normal saline, D5W or other compatible solutions.

Gemcitabine is sold under the trade name Gemzar® (Eli Lilly & Co., Indianapolis, IN). Gemzar is an antimetabolite related to cytarabine. Gemzar® is indicated for patients previously treated with 5-fluorouracil. Gemzar® is a pyrimidine analog that has a broad range of activity against solid tumors including but not limited to breast, ovarian, pancreatic, and lung carcinomas. It is believed to be incorporated into DNA of fast growing cancer cells, affecting replication. Gemzar® is a nucleoside analogue which disrupts DNA synthesis in S-phase cells and blocks the progression of cells through the G1/S phase boundary. Gemcitabine HCI is believed to be metabolized by nucleoside kinases to active diphosphate and triphosphate forms which inhibit ribonucleotide reductase and which competes with CTP for incorporation into DNA, respectively. Gemzar® is administered by intravenous injection (i.v.) or by other appropriate infusion techniques.

Gefitinib is sold under the tradename Iressa® (ZD 1839, Astra-Zeneca). Iressa is a 4-anilinoquinazoline and is believed to inhibit kinase acitivity of the epidermal growth factor regulator (EGFR). Mechanism of action studies seem to indicate that Iressa is an ATP-competitive inhibitor of EGFR and blocks autophosphorylation of the receptor when the receptor is stimulated by binding EGF or TGFα. Iressa is orally bioavailable and has demonstrated preclinal efficacy against tumor models that simultaneously express EGFR and one of its ligands, TGFα. Iressa has also been shown to inhibit the in vitro proliferation of cell lines that overexpress either EGFR or Her2. In clinical trials, Iressa has been maintained p.o. on a continuous daily schedule at up to 800 mg/day.

Doxorubicin (DOX) is sold under the tradename Adriamycin® (Adria). DOX is an anthracylcine that is believed to intercalate in DNA and interact with DNA Topoisomerase II to induce double-stranded DNA breaks. DOX exhibits a broad spectrum of anti-tumor efficacy. DOX is clinically administered intravenously on an intermittent schedule. The primary route of elimination of DOX is through the bile with no enterohepatic circulation. The dose-limiting acute toxicity of DOX is myelosuppresion. Other common, but not usually dose-limiting toxicities are gastrointestinal, alopecia, and local tissue damage/ulceration at the injection site due to extravasation of the drug.

Paclitaxel is sold under the tradename Taxol® by the Bristol-Myers Squibb Company. Paclitaxel (5β,20-Epoxy-1,2α,4,7β,10β,13α-hexahydroxytax-11-en-9-one 4,10-diacetate 2-benzoate 13-ester with (2R,3S)-N-benzoyl-3-phenyl-isoserine) has the empirical formula C₄₇H₅₁NO₁₄ and a molecular weight of 853.9. It is highly lipophilic in water. Paclitaxel is an antimicrotubule agent that promotes the assembly of microtubles from tubulin dimers and stabilizes microtubules by preventing depolymerization. While not bound by a theory, it is believed that this stability results in the inhibition in the normal dynamic reorganization of the microtubule network that is essential for vital interphase and mitotic cellular functions. Also, paclitaxel is believed to induce abnormal arrays or bundles of microtubules throughout the cell cycle and multiple asters of microtubules during mitosis. Paclitaxel is administered by intravenous injection or by other appropriate infusion techniques.

These and other cytotoxic/cytostatic agents can be administered in the conventional formulations and regimens in which they are known for use alone.

The aryl urea compound A can be administered orally, dermally, parenterally, by injection, by inhalation or spray, sublingually, rectally or vaginally in dosage unit formulations. The term 'administration by injection' includes intravenous, intraarticular, intramuscular, subcutaneous and parenteral injections, as well as use of infusion techniques. Dermal administration may include topical application or transdermal administration. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing the active materials in admixture with excipients suitable for the manufacture of aqueous suspensions may also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or *n*-propyl *p*-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in polyethyleneglycol, a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal or vaginal administration of the drug. These compositions can be prepared by mixing the drug with a suitable non-irritating excipient which is solid at ordinary temperatures but liquid at the rectal temperature or vaginal temperature and will therefore melt in the rectum or vagina to release the drug. Such materials include cocoa butter and polyethylene glycols.

Compounds of the invention may also be administrated transdermally using methods known to those skilled in the art (see, for example: Chien; "Transdermal Controlled Systemic Medications"; Marcel Dekker, Inc.; 1987. Lipp et al. WO94/04157 3Mar94). For example, a solution or suspension of an aryl urea compound in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of an aryl urea compound may be formulated into a lotion or salve.

Suitable solvents for processing transdermal delivery systems are known to those skilled in the art, and include dimethylsulfoxide, lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, ortrichlorofluoroethane. Suitable solvents may also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery systems are known to those skilled in the art, and include, for example, monohydroxy or polyhydroxy alcohols such as ethanol, propylene glycol or benzyl alcohol, saturated or unsaturated C₈₋C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, n-butyl, sec-butyl, isobutyl, tertbutyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures of one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene copolymers, and natural and synthetic rubbers. Cellulose ethers, derivatized polyethylenes, and silicates may also be used as matrix components. Additional additives, such as viscous resins or oils may be added to increase the viscosity of the matrix.

The invention also encompasses kits for treating mammalian cancers. Such kits can be used to treat a patient with a raf kinase stimulated cancer as well as cancers not stimulated through raf kinase. The kit can comprise a single pharmaceutical formulation containing aryl urea compound A and a cytotoxic or cytostatic agent. Alternatively, the kit can comprise aryl urea compound A and a cytotoxic or cytostatic agent in separate formulations. The kit can also include instructions for how to administer the compounds to a patient with cancer in need of treatment. The kit can be used to treat different cancer types which include but are not limited to colon, prostate, leukemia, melanoma, hepatocellular, renal, head and neck, glioma, lung, pancreatic, ovarian, and mammary.

It will be appreciated by those skilled in the art that the particular method of administration will depend on a variety of factors, all of which are routinely considered when administering therapeutics. It will also be understood, however, that the specific dose level for any given patient will depend upon a variety of factors, including, the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy. It will be further appreciated by one skilled in the art that the optimal course of treatment, i.e., the mode of treatment and the daily number of doses of aryl urea compound A given for a defined number of days, can be ascertained by those skilled in the art using conventional treatment tests.

The usefulness of a combination of aryl urea compound A with a cytotoxic or cytostatic agent is better than could have been expected from conventional knowledge of the effects of using either anticancer agent alone. For example, the combination therapy of aryl urea compound A with the cytotoxic agents irinotecan, gemcitabine, vinorelbine, or paclitaxel has produced at least additive anti-tumor efficacy compared with that produced by administration of either the aryl urea compound A or the cytotoxic agents administered alone. Generally, the use of cytotoxic and cytostatic agents in combination with aryl urea compound raf kinase inhibitor A will serve to (1) yield better efficacy in reducing the growth of a tumor or even eliminate the tumor as compared to administration of a single chemotherapeutic agent, (2) provide for the administration of lesser amounts of the administered chemotherapeutic agents, (3) provide for a chemotherapeutic treatment that is well tolerated in the patient with less deleterious pharmacological complications resulting from larger doses of single chemotherapies and certain other combined therapies, (4) provide for treating a broader spectrum of different cancer types in mammals, especially humans, (5) provide for a higher response rate among treated patients, (6) provide for a longer survival time among treated patients compared to standard chemotherapy treatments, (7) provide a longer time for tumor progression, and/or (8) yield efficacy and tolerability results at least as good as those of the agents used alone, compared to known instances where other cancer agent combinations produce antagonist effects.

The aryl urea compound A can be administered to a patient at a dosage which can range from about 0.1 to about 300 mg/Kg of total body weight. The daily dose for oral administration will preferably be from 0.1 to 300 mg/kg of total body weight. The daily dosage for administration by injection which includes intravenous, intramuscular, subcutaneous and parenteral injection as well as infusion techniques will preferably be from 0.1 to 300 mg/kg of total body weight. The daily vaginal dosage regime will preferably be from 0.1 to 300 mg/kg of total body weight. The daily topical dosage regimen will preferably be from 0.1 to 300 mg administered between one to four times daily. The transdermal concentration will preferably be that required to maintain a daily dose of from 1 to 300 mg/kg. For all the above mentioned routes of administration, the preferred dosage is 0.1 to 300 mg/kg. The daily inhalation dosage regimen will preferably be from 0.1 to 300 mg/kg of total body weight.

The cytotoxic or cytostatic agent can be administered to a patient at a dosage which can range from about 0.1 to about 300 mg/kg of total body weight. Also, the agents can also be administered in conventional amounts routinely used in cancer chemotherapy.

For both the aryl urea compound A and the cytotoxic or cytostatic agent, the administered dosage of the compound may be modified depending on any superior or unexpected results which may be obtained as routinely determined with this invention.

The aryl urea compound A can be administered orally, topically, parenterally, rectally, by inhalation, and by injection. Administration by injection includes intravenous, intramuscular, subcutaneous, and parenterally as well as by infusion techniques. The aryl urea compound A can be present in association with one or more non-toxic pharmaceutically acceptable carriers and if desired other active ingredients. A preferred route of administration for the aryl urea compound is oral administration.

The cytotoxic or cytostatic agent can be administered to a patient orally, topically, parenterally, rectally, by inhalation, and by injection. Administration by injection includes intravenous, intramuscular, subcutaneous, and parenterally as well as by infusion techniques. The agents can be administered by any of the conventional routes of administration for these compounds. The preferred route of administration for the cytotoxic/cytostatic agents using this invention is typically by injection which is the same route of administration used for the agent alone. Any of the cytotoxic or cytostatic agents can be administered in combination with aryl urea compound A by any of the mentioned routes of administration.

For administering the aryl urea compound A and the cytotoxic/cytostatic agent, by any of the routes of administration herein discussed, the aryl urea compound A can be administered simultaneously with the cytotoxic or cytostatic agent. This can be performed by administering a single formulation which contains both the aryl urea compound A and the cytotoxic/cytostatic agent or administering the aryl urea compound A and the cytotoxic/cytostatic agents in independent formulations at the same time to a patient.

Alternatively the aryl urea compound A can be administered in tandem with the cytotoxic/cytostatic agent. The aryl urea compound A can be administered prior to the cytotoxic/cytostatic agent. For example, the aryl urea compound A can be administered once or more times per day up to 28 consecutive days followed by administration of the cytotoxic or cytostatic agent. Also, the cytotoxic or cytostatic agent can be administered first followed by adminstration of the aryl urea compound A. The choice of sequence administration of the aryl urea compound A relative to the cytotoxic/cytostatic agent may vary for different agents. Also, the cytotoxic or cytostatic agent can be administered using any regimen which is conventionally used for these agents.

In another regimen of administration, the aryl urea compound A and the cytotoxic/cytostatic agent can be administered once or more times per day on the day of administration.

Any of the routes and regimens of administration may be modified depending on any superior or unexpected results which may be obtained as routinely determined with this invention.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius and, all parts and percentages are by weight, unless otherwise indicated.

### EXAMPLES

### Animals

Ncr *nul nu* female mice (Taconic Farms, Germantown, NY) were used for all in vivo studies invovling the DLD-1 and NCI-H460 tumor models. Female CB-17 SCID mice (Taconic Farms, Germantown, NY) were used for studies involving the Mia-PaCa-2 tumor model. The mice were housed and maintained within the Comparative Medicine Department at Bayer Corporation, West Haven, CT in accordance with Bayer IACUC, State, and Federal guidelines for the humane treatment and care of laboratory animals. Mice received food and water *ad libitum.*

### Compounds

Compound A (lot 9910071) was used in all studies. Compound A is a dry powder with a color ranging from white to ivory or light yellow. Compound A was stored in the dark until used.

Camptosar® (lot numbers 09FDY and 27FMR) was manufactured by Pharmacia-Upjohn and came supplied as a 20 mg/ml solution. It was stored at room temperature as indicated on the package insert.

Gemzar® (Gemcitabine HCl) was manufactured by Eli Lilly and Company and came supplied as a dry powder. It was stored at room temperature as indicated on the package insert.

Navelbine® (vinorelbine tartrate) was manufactured by GlaxoWellcome, came in as 10mg/ml solution. It was stored in 4°C as indicated on the package.

DOX (Doxorubicin HCl) was manufactured by Bedford Laboratories (lot 110033) and came supplied as a lyophilized red/orange powder. It was stored at 4°C and protected from light.

Gefitinib (ZD1839) (4-(3-chloro-4-fluoroanilino)7-methoxy-6-(3-morpholinopropoxy)quinazoline was synthesized by Albany Medical Research (Syracuse, NY). ZD 1839 was stored in the dark at room temperature until used.

### Vehicles

Cremophor EL/Ethanol (50:50) (Sigma Cremophor EL Cat.# C-5135; 500g, 95% Ethyl Alcohol), was prepared as a stock solution, wrapped with aluminum foil, and stored at room temperature. Compound A was formulated at 4-fold (4X) of the highest dose in this Cremophor EL/Ethanol (50:50) solution. This 4X stock solution was prepared fresh every three days. Final dosing solutions were prepared on the day of use by dilution to 1X with endotoxin screened distilled H₂O (GIBCO, Cat.# 15230-147) and mixed by vortexing immediately prior to dosing. Lower doses were prepared by dilution of the 1X solution with Cremophor EL/Ethanol/water (12.5:12.5:75). The vehicle for Camptosar® and Gemzar® was 0.9% saline and the vehicle for Navelbine® was D5W. All vehicles and compound solutions were stored at room temperature and wrapped in foil.

### Tumor Lines

The DLD-1 human colon carcinoma and the MiaPaCa-2 human pancreatic carcinoma were obtained from the American Type Tissue Culture Collection Repository. The MX-1 human mammary tumor was obtained from the NCI tumor repository. Tumors were maintained as a serial in vivo passage of s.c. fragments (3 x 3 mm) implanted in the flank using a 12 gauge trocar. A new generation of the passage was initiated every three or four weeks.

The NCI-H460 and A549 human non-small-cell lung carcinoma lines were obtained from the American Type Tissue Culture Collection Repository. The NCI-H460 cells were maintained and passaged in vitro using DMEM (GIBCO cat. # 11995-065: 500 mls) supplemented with 10% heat inactivated fetal bovine serum (JRH Biosciences cat.# 12106-500M), 2mM L-glutamine (GIBCO cat. # 25030-81), 10mM HEPES buffer (GIBCO cat # 15630-080) and penicillin-streptomycin (GIBCO cat. # 15140-122: 5 mls/50 mls DMEM). The A549 cells were maintained and passaged using RPMI 1640 media (GIBCO cat.# 11875-085: 1000ml) supplemented with 10% heat-inactivated fetal bovine serum (JRH Biosciences cat.# 12106-500M). All cells were maintained at 37°C and 5% CO₂ in a Fisher Scientific 610 CO₂ incubator.

### Tumor Xenograft Experiments

Female mice were implanted s.c. with DLD-1, MX-1 or Mia-PaCa-2 tumor fragments from an *in vivo* passage. Studies with the NCI-H460 and A549 cells were initiated by harvesting cells from an *in vitro* culture by adding Trypsin-EDTA (GIBCO cat#25200-056) for 2 minutes followed by centrifugation of the cells into a pellet and resuspension in HBSS (GIBCO cat# 14025-092) to a final cell count of 3-5 x 10⁷ viable cells/ml. A volume of 0.1ml of the cell suspension was injected s.c. in the right flank of each mouse. All treatment was initiated when all mice in the experiment had established tumors ranging in size from 100 to 150 mg. The general health of mice was monitored and mortality was recorded daily. Tumor dimensions and body weights were recorded twice a week starting with the first day of treatment. Animals were euthanized according to Bayer IACUC guidelines. Treatments producing greater than 20% lethality and/or 20% net body weight loss were considered 'toxic'.

Tumor weights were calculated using the equation (*l* x *w*²)/2, where *l* and *w* refer to the larger and smaller dimensions collected at each measurement. In each experiment, an evaluation endpoint was selected such that the median time for the tumors in the control group to attain that size was slightly greater than the duration of treatment. Anti-tumor efficacy was measured as the incidence of complete regressions (CR) defined as tumors that are reduced to below the limit of measurement (3 mm) in both length and width, partial regressions (PR) defined as tumors that are reduced by more than 50% but less than 100% of their initial size, and percent tumor growth suppression (%TGS). TGS is calculated by the equation [(T-C)/C] * 100, where T and C represent the times for the median tumors in the treated (T) and untreatred control (C) groups, respectively, to attain the evaluation size for that experiment.

### Results

### Combination of compound A and cytotoxidcytostatic agents

The most intensive combination chemotherapy anticipated in the clinical development of compound A for the treatment of cancer would involve daily administration of compound A administered throughout the period of time encompassing the intermittent administration of cytotoxic/cytostatic agents such as e.g., Camptosar®, Gemzar®, Navelbine®, or DOX that constitute the current clinical practice with each of these agents. In order to explore the potential interactions of these agents, we modeled this anticipated clinical schedule in our preclinical model by superimposing the schedules of the individual agents (qd x 9 for compound A and q4d x 3 for Camptosar®, Gemzar®, Navelbine®, or DOX) with both therapies in each experiment starting on the same day. An alternative schedule of combination chemotherapy would consist of daily administration of compound A throughout the period of time encompassing the continuous administration of cytostatic agents such as Iressa®. In order to explore the potential interactions of these agents, the preclinical model was established by superimposing the schedules of the individual agents (qd x 9 or 10 for both compound A and Iressa®). These schedules are termed 'Concurrent Therapy'. Each study consisted of an untreated control group of 10-20 animals and treated groups of 10 mice per group.

### Example 1

In the first study, Camptosar® was administered i.p at 40 mg/kg/dose. Compound A was administered p.o. on a qd x 9 schedule at 80 mg/kg/dose. All treatment was initiated on Day 7 post-implant when all animals had small but established DLD-1 human colon tumor xenografts averaging 108 mg in size. Control tumors grew progressively in all animals with an average doubling time of 4.4 days. The evaluation endpoint used to calculate the growth delay parameters was time to three mass doublings. The median time for the tumors in the untreated control group to attain that size was 10.4 days.

Camptosar® was well tolerated as a single agent with minimal weight loss and no lethality. The 40 mg/kg dose level produced a TGS of 71% with no complete or partial tumor regressions.

Compound A was also well tolerated as a single agent producing no significant weight loss and no lethality at 80 mg/kg/dose. Compound A produced a TGS of 100%.

There was no increase in weight loss and no lethality associated with the combination of Camptosar® with compound A. The anti-tumor efficacy of the concurrent therapy was at least additive producing a 229% TGS. This was associated with 3 PR's.

### Example 2

The second study evaluated Gemzar®, administered i.p at 120 mg/kg/dose on a q4d x 3 schedule and compound A, administered p.o. on a qd x 9 schedule at 40 mg/kg/dose. All treatment was initiated on Day 7 post-implant when all animals had small but established MiaPaCa-human pancreatic tumor xenografts averaging 108 mg in size. Control tumors grew progressively in all animals with an average doubling time of 4.1 days. The evaluation endpoint used to calculate the growth delay parameters was time to two mass doublings. The median time for the tumors in the untreated control group to attain that size was 5.8 days.

Gemzar® was well tolerated as a single agent with no weight loss and no lethality. This dose level produced a TGS of 154% with no complete or partial tumor regressions. Compound A was also well tolerated as a single agent producing no significant weight loss and no lethality at the 80 mg/kg dose level. Compound A produced TGS of 112%.There was no increase in weight loss and no lethality associated with the combination of Gemzar® with Compound A. The anti-tumor efficacy of the concurrent therapy of 120 mg/kg Gemzar and 40 mg/kg Compound A was at least additive producing a 222% TGS. This was associated with 2 PR's.

### Example 3

The third example demonstrates the effect of the combination of Compound A, administered p.o. on a qd x 9 schedule at 40 mg/kg/dose and Navelbine®, administered i.v. on a q4d x 3 schedule at 6.7 mg/kg/dose. All treatment was initiated on Day 6 post-implant when all animals had small but established NCI-H460 human non-small cell lung tumor xenografts averaging 100 mg in size. Control tumors grew progressively in all animals with an average doubling time of 3.1 days. The evaluation endpoint used to calculate the growth delay parameters was time to three mass doublings. The median time for the tumors in the untreated control group to attain that size was 7.4 days. The 6.7 mg/kg dose level of Navelbine was an approximate maximum tolerated dose producing an average 19% weight loss during the treatment period as a single agent. This was associated with a 32% TGS. Compound A was well tolerated with no significant weight loss and produced a TGS of 104%. The combination of these treatments was well tolerated with no lethality and an average weight loss of 14% (less than that produced by Navelbine alone). The antitumor efficacy of this combination was also approximately additive with a TGS of 133%.

### Example 4

The fourth example demonstrates the effect of the combination of Compound A, administered p.o. on a qd x 9 schedule at 40 mg/kg/dose and DOX, administered i.v. on a q^{4d} x 3 schedule at 4 mg/kg/dose. All treatments were initiated on Day 6 post-implant when all animals had small but established tumors averaging 66 mg in size. Control tumors grew progressively in all animals with an average doubling time of 3.7 days. The evaluation endpoint used to calculate the growth delay parameters was time to four mass doublings. The median time for the tumors in the untreated control group to attain that size was 14.5 days. The 4 mg/kg dose level of DOX was well tolerated producing an average 5% weight loss during the treatment period as a single agent. This was associated with a 43% TGS. Compound A was well tolerated with no significant weight loss and produced a TGS of 46%. The combination of these treatments was tolerated with no lethality and an average weight loss of 12%. The antitumor efficacy of this combination was also approximately additive with a TGS of 133%.

### Example 5

The fifth example demonstrates the effect of the combination of Compound A, administered p.o. on a qd x 9 schedule at 80 mg/kg/dose and Gefitinib (Iressa®), administered p.o. on a qd x 9 schedule at 150 mg/kg/dose. All treatment was initiated on Day 15 post-implant when all animals had small but established A549 human non-small cell lung tumor xenografts averaging 110 mg in size. Control tumors grew progressively in all animals with an average doubling time of 10.5 days. The evaluation endpoint used to calculate the growth delay parameters was time to one mass doubling.

The 150 mg/kg dose level of Iressa® was well tolerated producing no weight loss and no lethality during the treatment period as a single agent. This treatment was associated with a 101% TS and 1 PR. Compound A was also well tolerated as a single agent with no weight loss or lethality and produced a TGS of 218% with 1 CR and 2 PRs. The combination of these treatments was tolerated with one non-specific death out of 10 mice and an average 10% weight loss. The antitumor efficacy of this combination was approximately additive with a TGS of 314%. This treatment also produced 6 CR's and 3 PR's.

## Claims

1. A composition comprising an aryl urea compound which is a raf kinase inhibitor and (a) a cytotoxic agent or (b) a cytostatic agent (c) or a pharmaceutically acceptable salt of (a) or (b) wherein said aryl urea compound is a tosylate salt of N-(4-chloro-3-(trifluoromethyl)phenyl-N'-(4-(2-(N-methylcarbamoyl)-4-pyridyloxy)phenyl)urea.

2. The composition according to claim 1, in combination with one or more pharmaceutically acceptable carrier molecules.

3. The composition according to any of claim 1 or 2, wherein said cytotoxic agent or cytostatic agent is a DNA topoisomerase I inhibitor, a DNA topoisomerase II inhibitor, a DNA intercalator, an alkylating agent, a microtube disruptor, a hormone factor receptor antagonist/agonist or a growth factor receptor antagonist/agonist.

4. The composition according to any of claims 1 to 3, wherein said cytotoxic agent or cytostatic agent is irinotecan, vinorelbine, gemcitabine, geftinib, paclitaxel, taxotere, doxorubicin, cisplatin, carboplatin, BCNU, CCNU, DTIC, melphalan, cyclophosphamide, ara A, ara C, etoposide, vincristine, vinblastine, actinomycin D, 5-fluorouracil, methotrexate, herceptin, and mitomycin C.

5. The composition according to claim 1, wherein said cytotoxic agent is irinotecan.

6. The composition according to claim 1, wherein said cytotoxic agent is paclitaxel.

7. The composition according to claim 1, wherein said cytotoxic agent is vinorelbine.

8. The composition according to claim 1, wherein said cytotoxic agent is gemcitabine.

9. The composition according to claim 1, wherein said cytotoxic agent is doxorubicin.

10. The composition according to claim 1, wherein said cytostatic agent is geftinib.

11. The composition according to claim 1, wherein said composition is administered to a patient in need thereof at an oral, intramuscular, intravenous, subcutaneous, or parenteral dosage which can range from about 0.1 to about 300 mg/kg of total body weight.

12. The use of an aryl urea compound which is a raf kinase inhibitor and (a) a cytotoxic agent or (b) a cytostatic agent or (c) a pharmaceutically acceptable salt of (a) or (b) for the manufacture of a medicament for the treatment of cancer, wherein said aryl urea compound is a tosylate salt of N-(4-chloro-3-(trifluoromethyl)phenyl-N'-(4-(2-(N-methylcarbamoyl)-4-pyridyloxy)phenyl)urea.

13. The use of claim 12, wherein said cancer is colon, gastric, lung, pancreatic, ovarian, prostate, leukemia, melanoma, hepatocellular, renal, glioma, mammary, or head and neck cancer.

14. The use of claim 12, wherein said cytotoxic or cytostatic agent is a DNA topoisomerase I inhibitor, a DNA topoisomerase II inhibitor, a DNA intercalator, an alkylating agent, a microtube disruptor, a hormone factor receptor antagonist/agonist or a growth factor receptor antagonist/agonist.

15. The use of claim 12, wherein said cytotoxic or cytostatic agent is irinotecan, vinorelbine, gemcitabine, geftinib, paclitaxel, taxotere, doxorubicin, cisplatin, carboplatin, BCNU, CCNU, DTIC, melphalan, cyclophosphamide, ara A, ara C, etoposide, vincristine, vinblastine, actinomycin D, 5-fluorouracil, methotrexate, herceptin, and mitomycin C.

16. The use of claim 12, wherein said cytotoxic agent is irinotecan.

17. The use of claim 12, wherein said cytotoxic agent is paclitaxel.

18. The use of claim 12, wherein said cytotoxic agent is vinorelbine.

19. The use of claim 12, wherein said cytotoxic agent is gemcitabine.

20. The use of claim 12, wherein said cytotoxic agent is doxorubicin.

21. The use of claim 12, wherein said cytostatic agent is geftinib.

22. The use of claim 12, wherein said composition is administered in a therapeutically effective amount to a patient in need thereof by oral delivery or by intravenous injection or infusion.

23. The use of claim 12, wherein said composition is administered in a therapeutically effective amount to a patient in need thereof in the form of a tablet, a liquid, a topical gel, an inhaler or in the form of a sustained release composition.

24. The use of claim 12, wherein said composition is administered to a patient at an oral, intravenous, intramuscular, subcutaneous or parenteral dosage which can range from about 0.1 to about 300 mg/kg of total body weight.

25. A use of the composition of claim 1 for the manufacture of a medicament for inhibiting the proliferation of cancer cells in a patient comprising contacting said cancer cells with a pharmaceutical preparation comprising the composition of claim 1.

26. The use of an aryl urea compound which is a raf kinase inhibitor and (a) a cytotoxic agent or (b) a cytostatic agent or (c) a pharmaceutically acceptable salt of (a) or (b), wherein said aryl urea compound is a tosylate salt of N-(4-chloro-3-(trifluoromethyl)phenyl-N'-(4-(2-(N-methylcarbamoyl)-4-pyridyloxy)phenyl)urea, comprising an effective amount wherein said amounts are effective for treating tumors and have better efficacy in reducing tumor growth as compared to administration of either (a) or (b) or (c) alone for the manufacture of a medicament for the treatment of cancer.

## Patentansprüche

1. Zusammensetzung umfassend eine Arylharnstoff-Verbindung, die ein raf-Kinase Inhibitor ist, und (a) ein zytotoxisches Mittel oder (b) ein zytostatisches Mittel (c) oder ein pharmazeutisch annehmbares Salz von (a) oder (b), wobei die Arylharnstoff-Verbindung ein Tosylatsalz von N-(4-Chlor-3(trifluormethyl)phenyl-N'-(4-(2-(N-methylcarbamoyl)-4-pyridyloxy)phenyl) Harnstoff ist.

2. Zusammensetzung nach Anspruch 1, in Kombination mit einem oder mehreren pharmazeutisch annehmbaren Trägermolekülen.

3. Zusammensetzung nach einem der Ansprüche 1 oder 2, wobei das zytotoxische Mittel oder das zytostatische Mittel ein DNA Topoisomerase I Inhibitor, ein DNA Topoisomerase II Inhibitor, ein DNA Interkalator, ein Alkylierungsmittel, ein Mikrotubuli-spaltendes Mittel, ein Antagonist/Agonist eines Hormonfaktor-Rezeptors oder ein Antagonist/Agonist eines Wachstumsfaktor-Rezeptors ist.

4. Zusammensetzung nach einem der Ansprüche 1 bis 3, wobei das zytotoxische Mittel oder das zytostatische Mittel Irinotecan, Vinorelbin, Gemcitabin, Geftinib, Paclitaxel, Taxoter, Doxorubicin, Cisplatin, Carboplatin, BCNU, CCNU, DTIC, Melphalan, Cyclophosphamid, Ara A, Ara C, Etoposid, Vincristin, Vinblastin, Actinomycin D, 5-Fluoruracil, Methotrexat, Herceptin und Mitomycin C ist.

5. Zusammensetzung nach Anspruch 1, wobei das zytotoxische Mittel Irinotecan ist.

6. Zusammensetzung nach Anspruch 1, wobei das zytotoxische Mittel Paclitaxel ist.

7. Zusammensetzung nach Anspruch 1, wobei das zytotoxische Mittel Vinorelbin ist.

8. Zusammensetzung nach Anspruch 1, wobei das zytotoxische Mittel Gemcitabin ist.

9. Zusammensetzung nach Anspruch 1, wobei das zytotoxische Mittel Doxorubicin ist.

10. Zusammensetzung nach Anspruch 1, wobei das zytostatische Mittel Geftinib ist.

11. Zusammensetzung nach Anspruch 1, wobei die Zusammensetzung einem bedürftigen Patienten in einer oralen, intramuskulären, intravenösen, subkutanen oder parenteralen Dosis verabreicht wird, die in einem Bereich von circa 0,1 bis circa 300 mg/kg Gesamtkörpergewicht liegen kann.

12. Verwendung einer Arylharnstoff-Verbindung, die ein raf-Kinase Inhibitor ist, und (a) eines zytotoxischen Mittels oder (b) eines zytostatischen Mittels oder (c) eines pharmazeutisch annehmbaren Salzes von (a) oder (b) zur Herstellung eines Medikaments zur Behandlung von Krebs, wobei die Arylharnstoff-Verbindung ein Tosylatsalz von N-(4-Chlor-3-(trifluormethyl)phenyl-N'-(4-(2-(N-methylcarbamoyl)-4-pyridyloxy)phenyl) Harnstoff ist.

13. Verwendung nach Anspruch 12, wobei der Krebs Kolonkrebs, Magenkrebs, Lungenkrebs, Pankreaskrebs, Eierstockkrebs, Prostatakrebs, Leukämie, Melanom, Leberzellenkrebs, Nierenkrebs, Gliom, Brustkrebs oder Kopf- und Nackenkrebs ist.

14. Verwendung nach Anspruch 12, wobei das zytotoxische oder zytostatische Mittel ein DNA Topoisomerase I Inhibitor, ein DNA Topoisomerase II Inhibitor, ein DNA Interkalator, ein Alkylierungsmittel, ein Mikrotubuli-spaltendes Mittel, ein Antagonist/Agonist eines Hormonfaktor-Rezeptors oder ein Antagonist/Agonist eines Wachstumsfaktor-Rezeptors ist.

15. Verwendung nach Anspruch 12, wobei das zytotoxische oder zytostatische Mittel Irinotecan, Vinorelbin, Gemcitabin, Geftinib, Paclitaxel, Taxoter, Doxorubicin, Cisplatin, Carboplatin, BCNU, CCNU, DTIC, Melphalan, Cyclophosphamid, Ara A, Ara C, Etoposid, Vincristin, Vinblastin, Actinomycin D, 5-Fluoruracil, Methotrexat, Herceptin und Mitomycin C ist.

16. Verwendung nach Anspruch 12, wobei das zytotoxische Mittel Irinotecan ist.

17. Verwendung nach Anspruch 12, wobei das zytotoxische Mittel Paclitaxel ist.

18. Verwendung nach Anspruch 12, wobei das zytotoxische Mittel Vinorelbin ist.

19. Verwendung nach Anspruch 12, wobei das zytotoxische Mittel Gemcitabin ist.

20. Verwendung nach Anspruch 12, wobei das zytotoxische Mittel Doxorubicin ist.

21. Verwendung nach Anspruch 12, wobei das zytostatische Mittel Geftinib ist.

22. Verwendung nach Anspruch 12, wobei die Zusammensetzung einem bedürftigen Patienten in einer therapeutisch wirksamen Menge mittels oraler Verabreichung oder mittels intravenöser Injektion oder Infusion verabreicht wird.

23. Verwendung nach Anspruch 12, wobei die Zusammensetzung einem bedürftigen Patienten in einer therapeutisch wirksamen Menge in Form einer Tablette, einer Flüssigkeit, eines topischen Gels, mittels eines Inhalators oder in Form einer Zusammensetzung mit verzögerter Freisetzung verabreicht wird.

24. Verwendung nach Anspruch 12, wobei die Zusammensetzung einem Patienten in einer oralen, intravenösen, intramuskulären, subkutanen oder parenteralen Dosis verabreicht wird, die in einem Bereich von circa 0,1 bis circa 300 mg/kg Gesamtkörpergewicht liegen kann.

25. Verwendung der Zusammensetzung nach Anspruch 1 zur Herstellung eines Medikaments zur Inhibierung der Proliferation von Krebszellen in einem Patienten umfassend das in Kontakt bringen der Krebszellen mit einer pharmazeutischen Zubereitung, die die Zusammensetzung nach Anspruch 1 umfasst.

26. Verwendung einer Arylharnstoff-Verbindung, die ein raf-Kinase Inhibitor ist, und (a) eines zytotoxischen Mittels oder (b) eines zytostatischen Mittels oder (c) eines pharmazeutisch annehmbaren Salzes von (a) oder (b), wobei die Arylharnstoff-Verbindung ein Tosylatsalz von N-(4-Chlor-3(trifluormethyl)phenyl-N'-(4-(2-(N-methylcarbamoyl)-4-pyridyloxy)phenyl) Harnstoff ist, umfassend eine wirksame Menge, wobei diese Mengen zur Behandlung von Tumoren wirksam sind und, im Vergleich zu der Verabreichung von entweder (a) oder (b) oder (c) allein, eine bessere Wirksamkeit bei der Verringerung von Tumorwachstum aufweisen, zur Herstellung eines Medikaments zur Behandlung von Krebs.

## Revendications

1. Composition comprenant un composé de type urée aryle qui est un inhibiteur de kinase raf et (a) un agent cytotoxique ou (b) un agent cytostatique (c) ou un sel pharmaceutiquement acceptable de (a) ou (b) dans laquelle ledit composé de type urée aryle est un sel tosylate de la N-(4-chloro-3-(trifluorométhyl)phényl-N'-(4-(2-(N-méthylcarbamoyl)-4-pyridyloxy)phényl)urée.

2. Composition selon la revendication 1, en combinaison avec une ou plusieurs molécules de support pharmaceutiquement acceptables.

3. Composition selon l'une quelconque des revendications 1 ou 2, dans laquelle ledit agent cytotoxique ou agent cytostatique est un inhibiteur d'ADN topoisomérase I, un inhibiteur d'ADN topoisomérase II, un agent d'intercalation dans l'ADN, un agent alkylant, un désintégrateur de microtubes, un antagoniste/agoniste des récepteurs de facteurs hormonaux ou un antagoniste/agoniste des récepteurs de facteurs de croissance.

4. Composition selon l'une quelconque des revendications 1 à 3, dans laquelle ledit agent cytotoxique ou agent cytostatique est l'irinotécan, la vinorelbine, la gemcitabine, le géfitinib, le paclitaxel, le taxotère, la doxorubicine, le cisplatine, le carboplatine, le BCNU, le CCNU, le DTIC, le melphalan, le cyclophosphamide, l'ara A, l'ara C, l'étoposide, la vincristine, la vinblastine, l'actinomycine D, le 5-fluorouracile, le méthotrexate, l'herceptine, et la mitomycine C.

5. Composition selon la revendication 1, dans laquelle ledit agent cytotoxique est l'irinotécan.

6. Composition selon la revendication 1, dans laquelle ledit agent cytotoxique est le paclitaxel.

7. Composition selon la revendication 1, dans laquelle ledit agent cytotoxique est la vinorelbine.

8. Composition selon la revendication 1, dans laquelle ledit agent cytotoxique est la gemcitabine.

9. Composition selon la revendication 1, dans laquelle ledit agent cytotoxique est la doxorubicine.

10. Composition selon la revendication 1, dans laquelle ledit agent cytostatique est le géfitinib.

11. Composition selon la revendication 1, dans laquelle ladite composition est administrée à un patient en ayant besoin à une dose orale, intramusculaire, intraveineuse, sous-cutanée ou parentérale qui peut être située dans la plage d'environ 0,1 à environ 300 mg/kg de poids corporel total.

12. Utilisation d'un composé de type urée aryle qui est un inhibiteur de kinase raf et (a) d'un agent cytotoxique ou (b) d'un agent cytostatique ou (c) d'un sel pharmaceutiquement acceptable de (a) ou (b) pour la fabrication d'un médicament destiné au traitement du cancer, dans laquelle ledit composé de type urée aryle est un sel tosylate de la N-(4-chloro-3-(trifluorométhyl)phényl-N'-(4-(2-(N-méthyl-carbamoyl)-4-pyridyloxy)phényl)urée.

13. Utilisation selon la revendication 12, dans laquelle ledit cancer est un cancer du côlon, gastrique, du poumon, du pancréas, des ovaires, de la prostate, une leucémie, un mélanome, un cancer hépatocellulaire, rénal, un gliome, un cancer mammaire, ou un cancer de la tête et du cou.

14. Utilisation selon la revendication 12, dans laquelle ledit agent cytotoxique ou cytostatique est un inhibiteur d'ADN topoisomérase I, un inhibiteur d'ADN topoisomérase II, un agent d'intercalation dans l'ADN, un agent alkylant, un désintégrateur de microtubes, un antagoniste/agoniste des récepteurs de facteurs hormonaux ou un antagoniste/agoniste des récepteurs de facteurs de croissance.

15. Utilisation selon la revendication 12, dans laquelle ledit agent cytotoxique ou cytostatique est l'irinotécan, la vinorelbine, la gemcitabine, le géfitinib, le paclitaxel, le taxotère, la doxorubicine, le cisplatine, le carboplatine, le BCNU, le CCNU, le DTIC, le melphalan, le cyclophosphamide, l'ara A, l'ara C, l'étoposide, la vincristine, la vinblastine, l'actinomycine D, le 5-fluorouracile, le méthotrexate, l'herceptine, et la mitomycine C.

16. Utilisation selon la revendication 12, dans laquelle ledit agent cytotoxique est l'irinotécan.

17. Utilisation selon la revendication 12, dans laquelle ledit agent cytotoxique est le paclitaxel.

18. Utilisation selon la revendication 12, dans laquelle ledit agent cytotoxique est la vinorelbine.

19. Utilisation selon la revendication 12, dans laquelle ledit agent cytotoxique est la gemcitabine.

20. Utilisation selon la revendication 12, dans laquelle ledit agent cytotoxique est la doxorubicine.

21. Utilisation selon la revendication 12, dans laquelle ledit agent cytostatique est le géfitinib.

22. Utilisation selon la revendication 12, dans laquelle ladite composition est administrée en une quantité efficace d'un point de vue thérapeutique à un patient en ayant besoin par voie orale ou par injection ou perfusion intraveineuse.

23. Utilisation selon la revendication 12, dans laquelle ladite composition est administrée en une quantité efficace d'un point de vue thérapeutique à un patient en ayant besoin sous la forme d'un comprimé, d'un liquide, d'un gel topique, d'un inhalant ou sous la forme d'une composition à libération prolongée.

24. Utilisation selon la revendication 12, dans laquelle ladite composition est administrée à un patient a une dose orale, intraveineuse, intramusculaire, sous-cutanée ou parentérale qui peut être située dans la plage d'environ 0,1 à environ 300 mg/kg de poids total.

25. Utilisation de la composition selon la revendication 1 pour la fabrication d'un médicament destiné à inhiber la prolifération des cellules cancéreuses chez un patient comprenant la mise en contact desdites cellules cancéreuses avec une préparation pharmaceutique comprenant la composition selon la revendication 1.

26. Utilisation d'un composé de type urée aryle qui est un inhibiteur de kinase raf et (a) d'un agent cytotoxique ou (b) d'un agent cytostatique ou (c) d'un sel pharmaceutiquement acceptable de (a) ou (b), dans laquelle ledit composé de type urée aryle est un sel tosylate de la N-(4-chloro-3-(trifluorométhyl)phényl-N'-(4-(2-(N-méthylcarbamoyl)-4-pyridyloxy)phényl)urée, comprenant une quantité efficace dans laquelle lesdites quantités sont efficaces pour traiter des tumeurs et possèdent une meilleure efficacité pour réduire la croissance des tumeurs par rapport à la seule administration de soit (a) soit (b) soit (c) pour la fabrication d'un médicament destiné au traitement du cancer.
